# EUROPEAN PATENT APPLICATION

(11) **EP 3 351 289 A1**
(43) Date of publication of application: **25.07.2018**
(21) Application number: 17156697.9
(22) Date of filing: 17.02.2017
(51) Int. Cl.: A61N 1/36, A61N 1/362, A61N 1/365, A61B 5/053, A61B 5/04, A61B 5/00

(54) **SYSTEM AND METHOD FOR MEASURING EFFECTIVENESS OF AUTONOMIC NEUROSTIMULATION**

(30) Priority: 24.01.2017 US 201762449603 P
(71) Applicant: BIOTRONIK SE & Co. KG, 12359 Berlin (DE)
(72) Inventor: De VOIR, Christopher S., Tigard, OR 97223 (US); KIBLER, Andrew B., Lake Oswego, OR 97035 (US); MÜSSIG, Dirk, West Linn, OR 97068 (US)
(74) Representative: Keck, Hans-Georg

(57) **Abstract**

The invention relates to a system (1) for evaluating an efficacy of vagus nerve stimulation, wherein the system (1) comprises a neurostimulator (2) that is configured to perform vagus nerve stimulation, and a measuring means (3) for evaluating said efficacy based on at least one parameter (Z) that is indicative of a myocardial contractile state of the heart (H). Furthermore, the invention relates to a corresponding method.

## Description

The present invention relates to a system and a method for evaluating efficacy of vagus nerve stimulation (VNS).

Vagus nerve stimulation (VNS) is being studied for a variety of therapeutic applications, many of which take advantage of its increase in parasympathetic tone on the heart. Vagus nerve stimulation (VNS) has been shown to improve outcomes in cardiac ischemia, tachy-arhythmias, inflammatory diseases, and heart failure. A clear and rapid measure of stimulation efficacy is desired which allows stimulation titration and parameter optimization. Such a measure would also allow for improved system battery life and decreased side effects as a result of optimized stimulation.

Usually, measuring the efficacy of VNS with regard to an increase in cardioactive parasympathetic tone requires imaging systems that perform echocardiography, angiography, or plethysmography. Moreover, it is normally required to apply high stimulation amplitudes in order to obtain a measurable effect.

Furthermore, long-term measures of stimulation efficacy in the treatment of heart failure including blood vessels of inflammatory cytokine marker Pro-BNP, NYHA (New York Heart Association) heart failure class, ventricular diameter changes, or self-reported measures such as MLWHF (Minnesota Living With Heart Failure) score.

Reported methods of rapid cervical level VNS feedback include laryngeal activation measures via electromyography (EMG) or external accelerometer. These methods operate by observing side effects caused by activation of the recurrent laryngeal fibers contained in the cervical level vagal trunk, and are not a direct measure of the cardioprotective effect of parasympathetic activation.

Existing solutions for obtaining rapid measures of cardiac VNS effect require significant external or invasive equipment (echocardiography, RV plethysmography, angiography), or undesirably high VNS levels (heart rate). Other known solutions (laryngeal activation measures) are nonspecific and currently impractical for continuous use.

Long-term heart failure status measures of efficacy do not allow rapid therapy optimization and can be subjective.

Furthermore, US 8,939,904 B2 discloses a monitoring device for predicting cardiovascular anomalies, wherein the device may acquire a value change of a hemodynamic parameter, which occurs as a result of a detected value change of a state parameter.

Based on the above, the problem addressed by the present invention is to provide a system and a method for assessing efficacy of VNS.

This problem is solved by a system having the features of claim 1 and a method having the features of claim 9. Preferred embodiments of these aspects of the present invention are stated in the corresponding sub-claims and are described below.

According to claim 1, a system for evaluating an efficacy of vagus nerve stimulation is disclosed, wherein the system comprises a neurostimulator that is configured to perform vagus nerve stimulation, and a measuring means for evaluating said efficacy based on at least one parameter that is indicative of a myocardial contractile state (for example but not limited to inotropic and/or lusitropic states) of the (e.g. human) heart of the patient.

Particularly, both the neurostimulator and the measuring means are implantable into the body of the patient. Further, particularly, the measuring means is configured to communicate data to the neurostimulator. Particularly, the system may be configured to conduct bidirectional communication between the neurostimulator and the measuring means. The communication may be carried out via a line or wireless, for example based on radio frequency (RF), acoustic signals, optical signals, changes of a magnetic or electric field or the like. The data communicated between the neurostimulator and the measuring means may comprise measured values from the neurostimulator and the measuring means as well as information on stimulation parameters from the neurostimulator.

According to an embodiment of the system according to the present invention, said parameter is one of
- intracardiac impedance (Z),
- ventricular wall motion,
- heart sounds,
- low frequency fluid motion acoustic signals,
or a parameter derived therefrom, wherein the measuring means is configured to measure said parameter.

Furthermore, according to an embodiment of the system according to the present invention, the system according to claim 2, wherein said intracardiac impedance is measured in an unipolar manner, and wherein the measuring means comprises an electrode having a tip that is configured to be arranged at a location in the heart, preferably the apex of the right ventricle (RV) of the heart for measuring said intracardiac impedance. According to the invention an embodiments of the invention, the electrode tip may be arranged at any location in or around the heart, its chambers, tissue layers, vessels, or vicinity that would source a measure capable of evaluating VNS efficacy.

Particularly, the measuring means is configured to measure said impedance in a unipolar manner, which involves measuring the impedance between said tip and a counter electrode, wherein the counter electrode may be provided by a housing of said measuring means.

Particularly, according to an embodiment of the system according to the present invention, the neurostimulator may form a unit that is separate from (and particularly in communication with) the measuring means (i.e. these two components comprise separate housings). However, the neurostimulator and the measuring means may also be integrated into a single housing. The communication may be carried out via a line or wireless, for example based on radio frequency (RF), acoustic signals, optical signals, changes of a magnetic or electric field or the like.

Particularly, said intracardiac impedance signal obtained with this configuration is mainly determined by conductivity changes in the vicinity of the electrode tip, wherein such conductivity changes occur due to changes in the percentage of myocardial tissue volume to blood volume in the surrounding of the electrode tip during isovolumetric contraction and ejection. Therefore, said impedance signal is indicative of the geometrical changes of the myocardium during contraction. Thus, the intracardiac impedance may serve as a parameter or may be used to derive a parameter that correlates well with contractility of the heart. Since the contraction pattern of the heart is altered under sympathetic influence, the intracardiac impedance contains information about the autonomic nervous system (ANS).

Furthermore according to an embodiment of the system according to the present invention the neurostimulator is configured to perform vagus nerve stimulation (VNS) by activating parasympathetic ganglia in the heart, particularly either through stimulation of the vagus nerve or direct ganglia stimulation, particularly by means of electrical impulses generated by the neurostimulator and applied to said vagus nerve or ganglia by means of at least one or a plurality of stimulation electrodes of the neurostimulator.

Furthermore, according to an embodiment of the system according to the present invention, the system is configured to determine said parameter by means of the measuring means during diastole and/or systole of the cardiac cycle.

Particularly, in an embodiment, the system is configured to repeatedly determine said parameter during vagus nerve stimulation performed by the system and in the absence of vagus nerve stimulation and to compare the parameter obtained during vagus nerve stimulation with the parameter obtained in the absence of vagal nerve stimulation for evaluating said efficacy of the vagus nerve stimulation (e.g. the response of the heart's lusitropy and inotropy to said stimulation). Particularly, the relaxation of the myocardium around said electrode tip corresponds to lusitropy, whereas the contraction of the myocardium around said tip corresponds to inotropy. The comparison may be performed by evaluating the parameter with respect to at least one of a reference value, an upper and lower limit, a statistical moment, one or more direct or derived value from the same sensor at another time in the heart cycle, a direct or derived value from another sensor or sensors, or the state of a therapy device.

In this way, the measurements performed by the system provide real-time or trended implant-based feedback of the change in cardiac dynamics due to neuromodulation.

The comparative change of "neurostimulation on" vs. "neurostimulation off" of these parameters/measures provides a rapid assessment of neurostimulation efficacy.

Particularly, the vagus nerve stimulation is delivered by the system with a duty cycle 'on' period (vagus nerve stimulation present) of 10-30 seconds and an 'off period (vagus nerve stimulation absent) of 30 seconds to 5 minutes. Said measurements of the intracardiac impedance are preferably taken during VNS 'on' periods and compared against VNS 'off' periods, allowing 5-60 seconds for the VNS effect to wash out. Particularly, the measured parameters will provide information on cardiac function as well as allow guided titration of vagus nerve stimulation after implant for optimal efficacy. Such titration may include not only stimulation parameters by also a selection of the stimulation electrodes in case of a multi-electrode system (see also above).

Furthermore, according to an embodiment of the system according to the present invention, the system comprises an accelerometer configured to detect movements of the patient, wherein particularly the system is configured to conduct an algorithm which generates a measure for the patient's activity. Advantageously, the patient's activity trend will allow for long-term efficacy evaluation via its approximation of quality of life through activity.

Furthermore, according to an embodiment of the system according to the present invention, said derived parameter measured/determined by the system corresponds to one of:
- a time period over which the waveform of one of the intracardiac impedance (Z), ventricular wall motion, heart sounds or acoustic signals remains flat during the isovolumetric relaxation period, wherein particularly when lusitropy is improving due to the vagus nerve stimulation (VNS), the amount of time spent in this flat region will shorten in a pre-defined manner which is considered as a VNS having sufficient efficacy. The efficacy may be evaluated by any method suitable to evaluate a sample against a value direct or derived from a reference, limits, an expectation, or a device state. Appropriate scaling of such a result is dependent on the distribution of the data and clinical standards. - a time period between the closure of the aortic valve and the opening of the Mitral valve, which period of time is estimated by means of the first-order derivative of one of the measured intracardiac impedance (Z), ventricular wall motion, heart sounds or acoustic signals waveform, wherein particularly this time period between these two changes in valve state is reduced with effective VNS, wherein when said period of time is reduced in a pre-defined manner, the efficacy is considered as being sufficient.

For example, one approach to evaluate waveform flatness is to determine amplitude variation of the waveform. For instance, a waveform can be declared as flat when the amplitude has not varied for more than a certain percentage within a predetermined time. An exemplary process for defining flatness of an impedance waveform is given in the following: The impedance signal is sampled at a certain rate. A linear model is generated for the time/impedance pairs (such as z(t) ∼ m*t + b ) for t = 0, 1,.., n). An analysis of variance is applied and the resulting parameters are tested against the hypothesis that they differ significantly from the null hypothesis of a horizontal line (that is 'flat'). Since real physiologic signals even when sampling a 'flat' region will likely contain some non-zero offset and variance, these statistical moments can be tested with p-value and F-statistic to see if the 'flat' region varies significantly from an ideal horizontal line at a fixed direct current value.

Yet another aspect of the present invention relates to a method for obtaining a value indicating an efficacy of vagus nerve stimulation, particularly using a system according to the invention, wherein the value is obtained based on at least one parameter that is indicative of a myocardial contractile state of the heart.

Particularly, the vagus nerve stimulation itself is not a step or part of the claimed method, which is dedicated to measuring the effect of such a stimulation that has been applied in beforehand.

Preferably, according to an embodiment of the method according to the present invention, said parameter isone of:
- intracardiac impedance (Z),
- ventricular wall motion,
- heart sounds,
- low frequency fluid motion acoustic signals,
or a parameter derived therefrom, and wherein said parameter is measured.

Furthermore, according to an embodiment of the method according to the present invention, said intracardiac impedance is unipolar intracardiac impedance that is measured using an electrode having a tip that has been arranged at the right ventricular apex. Particularly, arranging said tip at the apex is not part of the claimed method, but is conducted in beforehand.

Particularly, according to an embodiment of the method according to the present invention, the unipolar impedance may be measured between said tip and a counter electrode, which counter electrode may be provided by a housing of a measuring means.

Furthermore, according to an embodiment of the method according to the present invention, said parameter is determined during diastole and/or systole of the cardiac cycle.

Furthermore, according to an embodiment of the method according to the present invention, said parameter is repeatedly determined during vagus nerve stimulation and in the absence of vagus nerve stimulation, and wherein the parameter obtained during vagus nerve stimulation is compared to the parameter obtained in the absence of vagus nerve stimulation for evaluating said efficacy.

Furthermore, according to an embodiment of the method according to the present invention, a movement of the body of the patient is detected in addition, and an activity measure of the patient is derived from said detected movements (see also above).

Furthermore, according to an embodiment of the method according to the present invention, said derived parameter corresponds to one of (see also above):
- a time period over which one of the intracardiac impedance (Z), ventricular wall motion, heart sounds or acoustic signals remains flat during the isovolumetric relaxation period, wherein particularly when lusitropy is improving due to the vagus nerve stimulation (VNS), the amount of time spent in this flat region will shorten;
- a time period between the closure of the aortic valve and the opening of the Mitral valve, which time period is estimated by means of the first-order derivative of one of the measured intracardiac impedance (Z), ventricular wall motion, heart sounds or acoustic signals, wherein the time period between these two changes in valve state is reduced with effective VNS.

Further features and advantages of the present invention and embodiments thereof shall be explained below with reference to the Figure and specific examples of the present invention, wherein
- Fig. 1: shows a system/method according to the present invention;
- Fig. 2: shows a heart suffering from compromised lusitropy due to increased sympathetic tone and the effect of VNS;
- Fig. 3: shows traces of a typical electrocardiogram, the corresponding intracardiac impedance and its first derivative, wherein the systole is highlighted;
- Fig. 4: shows traces of a typical electrocardiogram, the corresponding intracardiac impedance and its first derivative, wherein the diastole is highlighted;

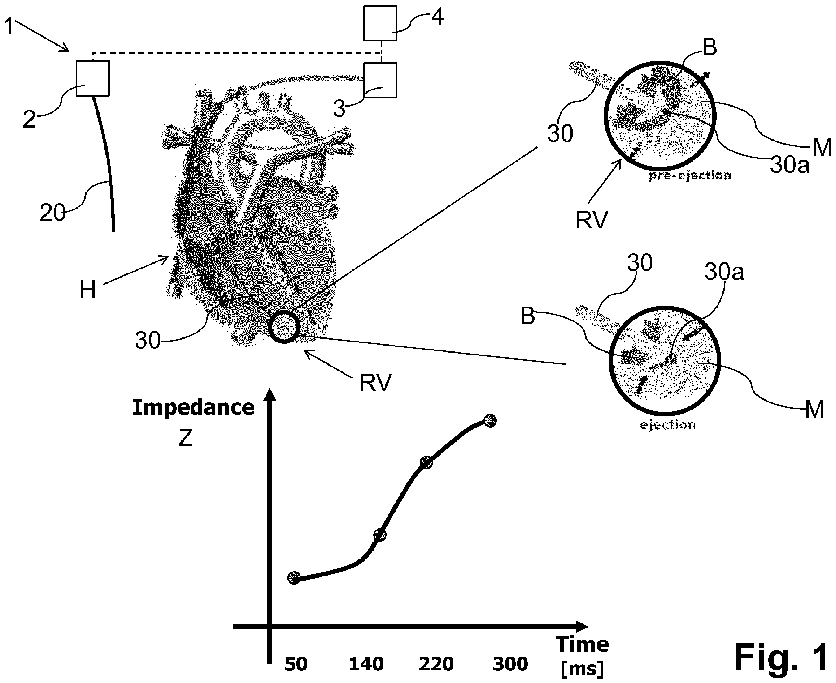

Fig. 1 shows a system 1 for evaluating an efficacy of vagus nerve stimulation VNS according to the present invention. The system 1 comprises a neurostimulator 2 that is configured to perform vagus nerve stimulation, and a measuring means 3 for evaluating said efficacy based on at least one parameter Z that is indicative of a myocardial contractile state of the heart H (e.g. as shown in the details of Fig. 1).

In addition, the proposed system 1 may incorporate an accelerometer 4 which measures patient activity and an algorithm which generates a patient activity trend. The patient activity trend will allow for long-term efficacy evaluation via its approximation of quality of life through activity.

Particularly, said parameter Z is an intracardiac impedance Z (or a parameter derived therefrom), wherein the measuring means 3 is configured to measure said intracardiac impedance Z, particularly by means of an electrode 30 in an unipolar configuration, wherein the electrode 30 comprises a tip 30a that is particularly arranged at the apex of the right ventricle RV. Using the unipolar electrode configuration for measuring impedance Z, the electrical path conducts through myocardium and blood, wherein the myocardium exhibits electrical impedance which is higher than blood. Consequently, the measured value of impedance Z depends on the relation of myocardium to blood within the electrical measurement path. That relation of myocardium to blood is depended on the cardiac contraction state, which is explained further in the following. Fig. 1 includes two detail illustrations which show the electrode 30 with electrode tip 30a in two different contraction states of the heart. In the 'pre-ejection' phase, the cardiac ventricles are filled with blood and the myocardium is relaxed. As a result, there is a comparatively high volume of blood B and low portion of myocardium in the vicinity of electrode tip 30a, resulting in a measured impedance Z which is low. In the 'ejection' phase of the heart, the blood is pumped out of the ventricles and the myocardium is in a contracted state. Consequently, a comparatively high portion of myocardium and small portion of blood surrounds electrode tip 30a, resulting in a measured impedance Z which is high.

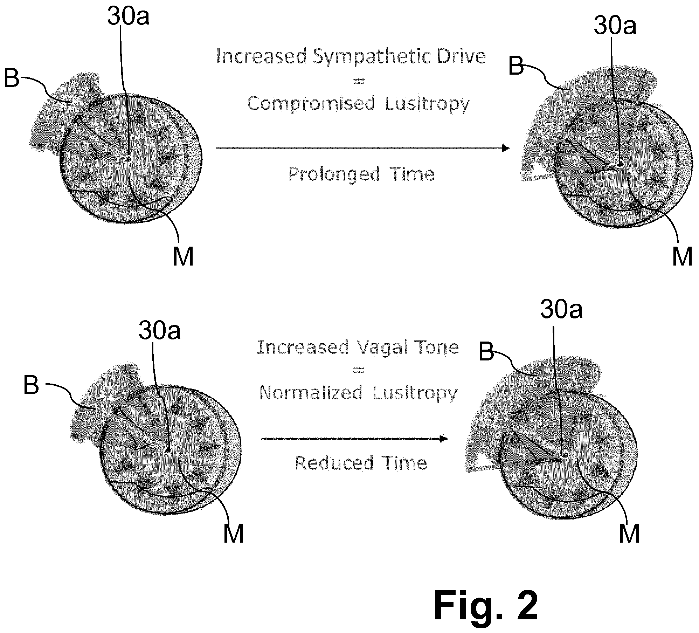

The ability of the myocardium to change from the contracted state to the relaxed state is called cardiac lusitropy. When a patient suffers from disturbed, i.e. increased sympathetic drive, cardiac lusitropy is compromised in a way which is illustrated in Fig. 2. The four images in Fig. 2 each show an intra-cardiac lead tip 30a embedded in the RV apex at the peak of contraction. The myocardium M contracts around the lead tip 30a, enveloping it in cardiac tissue which exhibits higher measurable electrical impedance than blood B. The upper half of Fig. 2 depicts schematically cardiac contraction behavior influenced by an increased sympathetic drive and how this is represented in the impedance measurements: Cardiac lusitropy is compromised in a way that the transition time for a change of the cardiac contraction state from high impedance (upper left image) to low impedance (upper right image) is prolonged, resulting in a prolonged time for the ventricle to relax and allowing passive phase diastolic pre-filling of the ventricle. In the lower half of Fig. 2, the effect of VNS therapy on a patient suffering from increased sympathetic tone is shown: Due to an increased vagal tone, the transition time from high impedance (lower left image) to low impedance (lower right image) is reduced, resulting in normalized cardiac lusitropy.

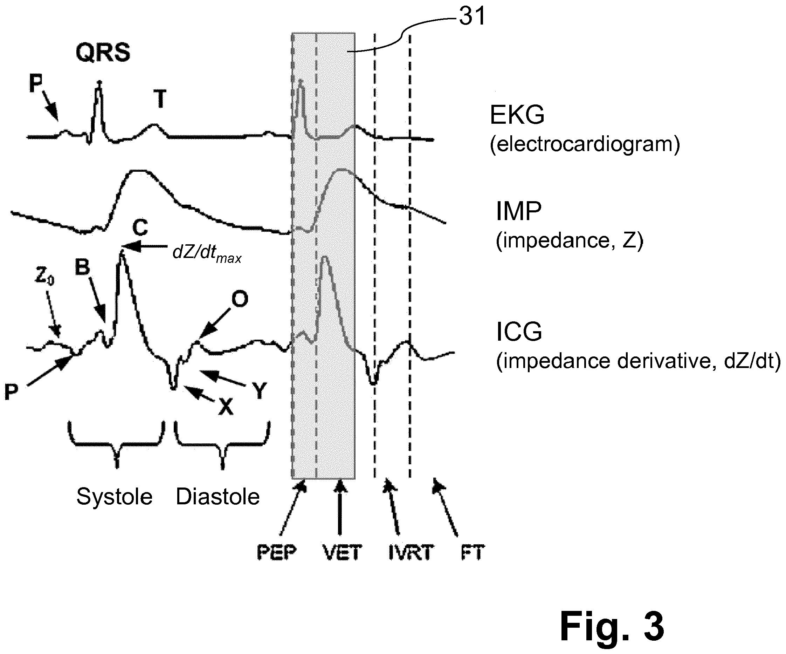

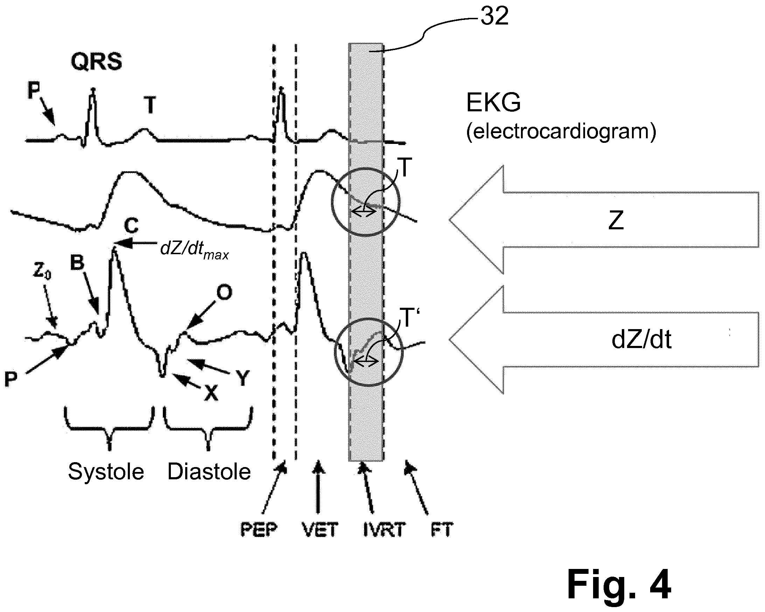

Fig. 3 shows a typical trace of an electrocardiogram of two cardiac cycles, the corresponding impedance Z measurements its first derivative dZ/dt. The highlighted area 31 marks a systolic phase of the heart. For evaluation of the impedance Z or a parameter derived therefrom according to the invention, the signals as shown can for instance be acquired with and without VNS, followed by signal analysis and comparison, for example with such signal parts acquired during diastole, as shown in highlighted area 32 of Fig. 4.

Particularly, the efficacy of VNS can be quantified in relation to how long (time period T) the continuous Impedance waveform Z remains flat during the isovolumetric relaxation period IRVT (see upper arrow in Fig. 4). If lusitropy is improving due to VNS, the amount of time spent in this flat region will shorten. An alternative indicator can be found in the first-order derivative of the intracardiac impedance dZ/dt which will reveal the closure of the aortic valve and the opening of the mitral valve. The length of time between these two changes in valve state is also reduced with effective VNS. One approach to evaluate waveform flatness is to determine amplitude variation of the waveform. For instance, a waveform can be declared as flat when the amplitude has not varied for more than a certain percentage within a predetermined time. An exemplary process for defining flatness of an impedance waveform is given in the following: The impedance signal is sampled at a certain rate. A linear model is generated for the time/impedance pairs (such as z(t) ∼ m*t + b) for t = 0, 1,.., n). An analysis of variance is applied and the resulting parameters are tested against the hypothesis that they differ significantly from the null hypothesis of a horizontal line (that is 'flat'). Since real physiologic signals even when sampling a 'flat' region will likely contain some non-zero offset and variance, these statistical moments can be tested with p-value and F-statistic to see if the 'flat' region varies significantly from an ideal horizontal line at a fixed direct current value.

Additional methods of establishing VNS efficacy in improving cardiac function include differential estimates of inotropy and lusitropy via measurements of the intracardiac impedance at systole and diastole, respectively. In cases of both heart failure with reduced ejection fraction (HFrEF, systolic HF) and preserved ejection fraction (HFpEF, diastolic HF) the differential measure of dZ/dt at these time points improves with therapy and improved cardiac function.

Particularly, according to the invention, VNS is delivered by the system 1 with a duty cycle 'on' period of 10-30 seconds and an 'off' period of 30 seconds to 5 minutes. Measurements of the parameter according to the invention are taken during VNS 'on' periods and compared against VNS 'off' periods, allowing 5-60 seconds for the VNS effect to wash out, provide a rapid efficacy estimate of VNS during normal device operation as well as the initial VNS up-titration period after implant.

## Claims

1. A system (1) for evaluating an efficacy of vagus nerve stimulation, wherein the system (1) comprises a neurostimulator (2) that is configured to perform vagus nerve stimulation, and a measuring means (3) for evaluating said efficacy based on at least one parameter that is indicative of a myocardial contractile state of the heart.

2. The system of claim 1, wherein said parameter is one of:
• intracardiac impedance (Z),
• ventricular wall motion,
• heart sounds,
• low frequency fluid motion acoustic signals,
or a parameter derived therefrom wherein the measuring means is configured to measure saidparameter.

3. The system according to claim 2, wherein said intracardiac impedance (Z) is measured in a unipolar manner, and wherein the measuring means (3) comprises an electrode (30) having a tip (30a) that is configured to be arranged at a location in the heart, preferably the apex of the right ventricle (RV) of the heart for measuring said intracardiac impedance.

4. The system according to one of the preceding claims, wherein the neurostimulator (2) is configured to activate parasympathetic ganglia in the heart (H), wherein for activating said ganglia the neurostimulator (2) is configured to generate electrical impulses and to apply them via at least one or a plurality of stimulation electrodes (20).

5. The system according to one of the preceding claims, wherein the system (1) is configured to determine said parameter by means of the measuring means (3) during diastole and/or systole of the cardiac cycle.

6. The system according to one of the preceding claims, wherein the system (1) is configured to repeatedly determine said parameter during vagus nerve stimulation and in the absence of vagus nerve stimulation and to compare the parameter obtained during vagus nerve stimulation with the parameter obtained in the absence of vagus nerve stimulation for evaluating said efficacy, wherein the comparison may be performed by evaluating the parameter with respect to at least one of a reference value, an upper and lower limit, a statistical moment, one or more direct or derived value from the same sensor at another time in the heart cycle, a direct or derived value from another sensor or sensors, or the state of a therapy device.

7. The system according to one of the preceding claims, wherein the system (1) comprises an accelerometer (4) configured to detect movements of the patient.

8. The system according to one of the claims 2 to 7, wherein said derived parameter corresponds to one of:
- a time period (T) the waveform of one of the intracardiac impedance (Z), ventricular wall motion, heart sounds or acoustic signals remains flat during the isovolumetric relaxation period;
- a time period (T') between the closure of the aortic valve and the opening of the Mitral valve of the heart (H), which time period (T') is estimated by means of the first-order derivative of one of the measured intracardiac impedance (Z), ventricular wall motion, heart sounds or acoustic signals waveform.

9. A method for obtaining a value indicating an efficacy of vagus nerve stimulation, particularly using a system (1) according to one of the preceding claims, wherein said value is obtained based on at least one parameter that is indicative of a myocardial contractile state of the heart (H).

10. The method of claim 9, wherein said parameter (Z) is wherein said parameter is one of:
• intracardiac impedance (Z),
• ventricular wall motion,
• heart sounds,
• low frequency fluid motion acoustic signals,
or a parameter derived therefrom, and wherein said parameter is measured.

11. The method according to claim 10, wherein said intracardiac impedance (Z) is an unipolar intracardiac impedance (Z) that is measured using an electrode (30) having a tip (30a) that has been arranged at the apex of the right ventricle (RV).

12. The method according to one of the claims 9 to 11, wherein said parameter (Z) is determined during diastole and/or systole of the cardiac cycle.

13. The method according to one of the claims 9 to 12, wherein said parameter is repeatedly determined during vagus nerve stimulation and in the absence of vagus nerve stimulation, and wherein the parameter obtained during vagus nerve stimulation is compared to the parameter obtained in the absence of vagus nerve stimulation for evaluating said efficacy.

14. The method according to one of the claims 9 to 13, wherein additionally a movement of the patient is detected and an activity measure of the patient is derived from said detected movements.

15. The method according to one of the claims 10 to 14, wherein said derived parameter corresponds to one of:
- a time period (T) the waveform of one of the intracardiac impedance (Z), ventricular wall motion, heart sounds or acoustic signals remains flat during the isovolumetric relaxation period;
- a time period (T') between the closure of the aortic valve and the opening of the Mitral valve of the heart (H), which time period (T') is estimated by means of the first-order derivative of one of the measured intracardiac impedance (Z), ventricular wall motion, heart sounds or acoustic signals waveform.
